(19) Europäisches Patentamt
European Patent Office
Office européen des brevets

(11) EP 0 796 657 B1

(12) EUROPEAN PATENT SPECIFICATION

(45) Date of publication and mention of the grant of the patent:
**16.01.2002 Bulletin 2002/03**

(51) Int Cl.[7]: **C07C 2/58**, C07C 2/62, B01J 31/02, B01J 27/12

(21) Application number: **97101110.1**

(22) Date of filing: **18.06.1992**

(54) **Alkylation catalyst containing hydrofluoric acid and a sulfone**

Fluorwasserstoffsäure und Sulfone enthaltender Alkylierungskatalysator

Catalyseur d'alkylation contenant de l'acide fluorhydrique et une sulfone

(84) Designated Contracting States:
**DE GB IT NL**

(30) Priority: **21.06.1991 US 719879**
**31.03.1992 US 860966**

(43) Date of publication of application:
**24.09.1997 Bulletin 1997/39**

(62) Document number(s) of the earlier application(s) in accordance with Art. 76 EPC:
**92914998.7 / 0 593 614**

(73) Proprietor: **PHILLIPS PETROLEUM COMPANY**
**Bartlesville Oklahoma (US)**

(72) Inventors:
- **Chalam, Manoj**
  **Mantua, New Jersey 08051 (US)**
- **Child, Johnathan Edward**
  **Sewell, New Jersey 08080 (US)**
- **Del Rossi, Kenneth Joseph**
  **Woodbury, New Jersey 08096 (US)**
- **Huss, Albin, Jr.**
  **Chaddsford, Pennsylvania 19317 (US)**
- **Kurtas, Robert Steve**
  **Ranch Palos Verdes, California 90274 (US)**
- **Schatz, Claus Wilhelm**
  **Princeton, New Jersey 08540 (US)**

(74) Representative:
**Dost, Wolfgang, Dr.rer.nat., Dipl.-Chem. et al**
**Patent- und Rechtsanwälte Bardehle .**
**Pagenberg . Dost . Altenburg . Geissler .**
**Isenbruck Postfach 86 06 20**
**81633 München (DE)**

(56) References cited:
**US-A- 3 795 712** **US-A- 4 024 203**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to an HF-containing isoparaffin-olefin alkylation catalyst. More particularly, the invention relates to the increase of rainout from a hydrofluoric acid- containing cloud formed from the escape of a hydrofluoric acid containing isoparaffin-olefin alkylation catalyst from its containment facility.

Industrial chemical plants, including petroleum refineries, often store and process hazardous fluids at elevated pressures. The petroleum refining industry in particular has compiled a record of safe, reliable unit operation through careful attention to maintenance and operator training. By minimizing accidents, the industry has successfully maintained a first line of protection against the hazards associated with the accidental release of corrosive and toxic fluids.

The second line of protection is necessarily tailored to the characteristics of the individual fluids. For example, the principal hazard associated with releasing a light aliphatic hydrocarbon (e.g., propane) would be ignition and explosion of the dense gas cloud. In contrast, releasing a toxic but non-flammable gas would pose various safety and environmental hazards other than explosion.

With the expansion of population centers near the battery limits of numerous petroleum refineries, concerns have arisen regarding the ability of presently used containment methods to prevent migration of aerosol or vapor clouds containing toxic substances (e.g., hydrofluoric acid) outside the refinery in the unlikely event of an accidental release.

Further, the importance of hydrofluoric acid (HF) catalyzed isoparaffin-olefin alkylation continues to grow as gasolines are reformulated to meet increasingly stringent air quality regulations. Specifically, the addition of HF alkylate to the gasoline pool can help compensate for the octane lost by removing alkyl-substituted aromatics.

Alkylation is a reaction in which an alkyl group is added to an organic molecule. Thus an isoparaffin can be reacted with an olefin to provide an isoparaffin of higher molecular weight. Industrially, the concept depends on the reaction of a $C_2$ to $C_5$ olefin with isobutane in the presence of an acidic catalyst producing a so-called alkylate. This alkylate is a valuable blending component in the manufacture of gasolines due not only to its high octane rating but also to its sensitivity to octane-enhancing additives.

Industrial alkylation processes have historically used concentrated hydrofluoric or sulfuric acid catalysts under relatively low temperature conditions. Acid strength is preferably maintained at 88 to 94 weight percent by the continuous addition of fresh acid and the continuous withdrawal of spent acid. As used herein, the term "concentrated hydrofluoric acid" refers to a liquid containing at least about 85 weight percent HF.

Hydrofluoric and sulfuric acid alkylation processes share inherent drawbacks including environmental and safety concerns, acid consumption, and sludge disposal. For a general discussion of sulfuric acid alkylation, see the series of three articles by L.F. Albright et al., "Alkylation of Isobutane with $C_4$ Olefins", 27 Ind. Eng. Chem. Res., 381-397, (1988). For a survey of hydrofluoric acid catalyzed alkylation, see 1 Handbook of Petroleum Refining Processes 23-28 (R.A. Meyers, ed., 1986).

Hydrogen fluoride, or hydrofluoric acid (HF) is highly toxic and corrosive. However, it is used as a catalyst in isomerization, condensation, polymerization and hydrolysis reactions. The petroleum industry used anhydrous hydrogen fluoride primarily as a liquid catalyst for alkylation of olefinic hydrocarbons to produce alkylate for increasing the octane number of gasoline. Years of experience in its manufacture and use have shown that HF can be handled safely, provided the hazards are recognized and precautions taken. Though many safety precautions are taken to prevent leaks, massive or catastrophic leaks are feared primarily because the anhydrous acid will fume on escape creating a vapor cloud that can be spread for some distance. Previous workers in this field approached this problem from the standpoint of containing or neutralizing the HF cloud in the event of an accidental release.

U.S. Patents 4,938,935 and 4,985,220 as well as U.S. Patent 4,938,936 to Yan teach various methods for containing and/or neutralizing HF acid clouds following accidental releases.

But it would be particularly desirable to provide an additive which decreases the cloud forming tendency of HF without compromising its activity as an isoparaffin-olefin alkylation catalyst. Solvents and complexing agents for hydrofluoric acid have, in the past, been disclosed for various purposes as noted in the following references.

U.S. Patent 2,615,908 teaches thioether-HF-copper complex compounds and a method for preparing the same. Potential uses for the thioether-HF-copper composition compounds are listed from column 6, line 55 through column 8 at line 3. The method is said to be useful for purifying HF-containing vent gases from an industrial HF alkylation plant. See column 7, lines 10-24.

U.S. Patent 3,531,546 discloses a HF-$CO_2$ catalyst composition which is said to be useful for alkylation as well as olefin isomerization.

U.S. Patent 3,795,712 relates to acid catalysts comprising a Lewis acid, a Bronsted acid, and a sulfone of the formula R-$SO_2$-R', where R and R' are each separately a monovalent radical containing from 1 to 8 carbon atoms or form together a divalent radical having from 3 to 12 carbon atoms.

U.S. Patent 3,856,764 teaches an olefin polymerization catalyst comprising (1) at least one organoaluminum compound, (2) at least one nickel compound selected from the class consisting of nickel salts of carboxylic acids, organic complex compounds of nickel, or nickel tetracarbonyl and (3) at least one hydrogen fluoride complex prepared

by complexing hydrogen fluoride with a member of the class consisting of ketones, ethers, esters, alcohols, nitriles, and water.

U.S. Patent 4,025,577 and 4,094,924 report the use of alkylation catalyst compositions containing HF, a metal halide, and sulfolane.

Promoters such as alcohols, thiols, water, ethers, thioethers, sulfonic acids, and carboxylic acids are disclosed in combination with strong Bronsted acids such as HF, fluorosulfonic and trihalomethanesulfonic acids in U.S. Patent 3,778,489. The promoters are said to modify the activity of the strong Bronsted acids for alkylation.

U.S. Patent 5,073,674 relates to a an alkylation process which uses a liquid onium polyhydrogen fluoride complex as reaction medium and catalyst. The Olah patent states that the disclosed complexing method reduces the volatility of the hydrofluoric acid.

The present invention allows to increase rainout from an autorefrigerated vaporous cloud containing hydrofluoric acid formed from the escape of hydrofluoric acid from its containment facility comprising admixing from about 50 to about 80 weight percent hydrofluoric acid with from about 20 to about 50 weight percent of at least one member selected from the group consisting of a sulfone having the formula $R-SO_2-R'$, wherein R and R' are the same or are different, and are selected from the group consisting of halides, alkyl groups, aromatic groups, alkylhalide groups, or arylhalide groups containing from about 1 to about 20 carbon atoms and a sulfone having the formula $R-SO_2$, wherein R is a bidentate alkyl group, an aromatic group, an alkylhalide group, or an arylhalide group containing from about 1 to about 20 carbon atoms.

Figure 1a is a plot of TMP/DMH ratio (trimethypentane/ dimethylhexane) as a function of weight percent sulfolane in HF for isoparaffin-olefin alkylation at 80°F (27°C), 175 psig (1,31 MPa), 10/1 isobutane/butene ratio and olefin WHSV of 0.3 $hr^{-1}$.

Figure 1b is a plot of $C_9$+ byproduct in the alkylate product as a function of weight percent sulfolane in HF under the conditions set forth with reference to Figure la.

Figure lc is a plot of the MON (Motor Octane Number) of the $C_5$+ portion of the alkylate product produced by the isoparaffin-olefin reaction (described above with reference to Figure la) as a function of weight percent sulfolane in HF.

Figure 2 is a plot of vapor pressure at 76°F (24°C) as a function of weight percent sulfolane in HF.

Figure 3 is a plot of percent rainout (weight) as a function of vapor pressure for neat sulfolane, neat HF, and mixtures of HF and sulfolane containing from 20 to 80 percent sulfolane (weight).

The present invention allows to increase the rainout from an autorefrigerated vaporous cloud containing hydrofluoric acid formed from the escape of hydrofluoric acid from its containment facility. The invention employs a sulfone additive which is compatible with hydrofluoric acid for use in a commercial isoparaffin-olefin alkylation process. Accordingly, the invention may comprise admixing the sulfone with the hydrofluoric acid and circulating the admixture as a liquid catalyst in a commercial isoparaffin-olefin alkylation process unit. Alternatively, the sulfone may be added to the hydrofluoric acid inventory in the unlikely event of a hydrofluoric acid escape from the containment facility.

Feedstocks useful in the isoparaffin-olefin alkylation process include at least one isoparaffin and at least one olefin. The isoparaffin reactant used in the present alkylation process has from about 4 to about 8 carbon atoms. Representative examples of such isoparaffins include isobutane, isopentane, 3-methylhexane, 2-methylhexane, 2,3-dimethylbutane and 2,4-dimethylhexane.

The olefin component of the feedstock includes at least one olefin having from 2 to 12 carbon atoms. Representative examples of such olefins include butene-2, isobutylene, butene-1, propylene, ethylene, hexene, octene, pentene, and heptene, merely to name a few. The preferred olefins include the $C_4$ olefins, for example, butene-1, butene-2, isobutylene, or a mixture of one or more of these $C_4$ olefins, with butene-2 being the most preferred. Suitable feedstocks for the process of the present invention are described in U.S. Patent 3,862,258 to Huang et al. at column 3, lines 44-56.

The molar ratio of isoparaffin to olefin is generally from about 1:1 to about 100:1, preferably from about 1:1 to about 50:1, and more preferably from about 5:1 to about 20:1.

The catalyst complex of the present invention comprises from about 50 to about 80 weight hydrofluoric acid and the halogenated sulfonic acids together with from about 20 to about.50 weight percent of a sulfone having the formula $R-SO_2-R'$, wherein R and R' are the same or are different, and are selected from the group consisting of halides, alkyl groups, aromatic groups, alkylhalide groups, or arylhalide groups containing from about 1 to about 20 carbon atoms. The sulfone may alternatively have the formula $R-SO_2$, wherein R is a bidentate alkyl group, an aromatic group, an alkylhalide group, or an arylhalide group containing from about 1 to about 20 carbon atoms.

Of these useful sulfones, sulfolane (tetramethylene sulfone) formula:

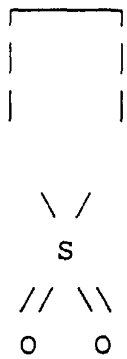

isparticularly preferred. More specifically, it has been found that a particular range of sulfone concentrations provide useful levels of isoparaffin-olefin alkylation activity while effectively suppressing the cloud forming tendency of the strong acid.

In a preferred embodiment, the catalyst composition comprises from about 20 to about 40 weight percent sulfolane and from about 60 to about 80 weight percent HF. The most preferred catalyst composition comprises from about 20 to about 30 weight percent sulfolane together with from about 70 to about 80 weight percent HF.

The catalyst composition of the present invention may be readily substituted for the concentrated hydrofluoric acid catalyst in an existing hydrofluoric acid alkylation process unit, for example, a riser reactor alkylation process unit, without substantial equipment modifications, Accordingly, the conversion conditions for the process of the present invention resemble those of typical commercial hydrofluoric acid alkylation processes.

The present alkylation process is suitably conducted at temperatures of from about 0 (-18°C) to about 150°F (66°C), preferably from about 50 (10°C) to about 150°F (66°C), and more preferably from about 70 (21°C) to about 110°F (43°C). Pressure is maintained to ensure a liquid phase in the alkylation reaction zone. Pressures typically range from about 20 (0,24 MPa) to about 1200 psig (8,4 MPa), preferably from about 50 (0,45 MPa) to about 500 psig (3,5 MPa). The reaction zone is preferably free from added hydrogen. Olefin feed rates generally range from about 0.01 to about 50 WHSV and more preferably from about 0.5 to about 20 $hr^{-1}$ WHSV. The mixed isoparaffin-olefin reactants may be contacted with the catalyst composition of the invention in any suitable reaction vessel, examples of which include stirred-tank reactors as well as riser-type reactors. Contact time for the mixed isoparaffin-olefin feed and the catalyst composition of the invention typically are within the range of from about 0.1 second to about 50 seconds, and are preferably from about 8 seconds to about 25 seconds.

The relative amounts of catalyst and reactants are defined herein by the acid-to-oil ratio. The volumetric acid-to-oil ratio (as used herein) is the ratio of the sum of the volumes of ASO (acid soluble oil), acid, and sulfone to the total isoparaffin and olefin reactor feed. The volumetric acid-to-oil ratio typically falls within the range of about 0.1:1 to about 10:1, preferably from about 0.1:1 to about 5:1. Contact time between the catalyst and the isoparaffin-olefin feedstock typically ranges from about 1 to about 50 seconds, preferably from about 8 to about 25 seconds.

The sulfone component of the alkylation catalyst composition may be added by injection directly into the alkylation process unit, or may be mixed with the hydrocarbon charge, or may be mixed with the fresh and/or the circulating acid catalyst component, or with a stream of mixed acid/additive catalyst. Downstream from the alkylation reaction zone, the sulfone is preferably separated from the alkylate product stream, mixed with fresh and/or circulating acid and/or circulating acid/additive catalyst mixture, and recycled to the alkylation reaction zone. The particular separation technique selected, however, depends upon the characteristics of the selected sulfone.

The sulfone may partition between the acid and the alkylate-containing hydrocarbon reactor effluent, or may remain in either the hydrocarbon or the acid phase, or may form a third discrete phase, depending upon the characteristics of the selected sulfone. If the boiling point of the selected sulfone does not overlap major hydrocarbon products, distillation is preferred to separate and recycle the sulfone. Higher boiling (e.g. >200°C) sulfones may require extraction (for example, liquid-liquid solvent extraction) to be efficiently recovered from alkylation byproducts such as ASO (acid soluble oil).

Examples

The following Examples demonstrate the effectiveness of the sulfone-containing catalyst composition of the invention for catalyzing isoparaffin-olefin alkylation. Example 1 demonstrates the well-known effectiveness of anhydrous HF as an isoparaffin-olefin alkylation catalyst and is presented for comparison to evaluate the effectiveness of the alkylation catalyst composition of the present invention (Examples 2-4).

Example 1 - Comparative

Anhydrous HF (40 grams, obtained from Matheson Chemical Company of Bridgeport, New Jersey) was condensed into a clean, dry autoclave (1000 cc). Isobutane (100 grams) was added, and the autoclave was stirred at 1500 rpm. The autoclave was brought to room temperature (22°C, 71°F) and pressurized to 100 psig (0,79 MPa). A premixed 10:1 weight:weight mixture of isobutane:2-butene feed (obtained from Matheson Chemical Company) was added at a rate of 250 cc/hour for 2 hours under autogeneous pressure for a total isobutane:2-butene charge of 500 cc. A 10-15°F (5-8°C) temperature rise was observed during feed addition resulting in an average reaction temperature of 27-30°C (80-85°F). The autoclave was sampled (300 cc) immediately after feed addition was complete. The sample was flashed at room temperature and quenched with a chilled water trap. Samples of the liquid and gas products were analyzed by capillary GC (60m DB-1 column). The results of Example 1 are shown in the Table A below.

Examples 2-4

The following procedure was followed for Examples 2-4. In a typical experiment, 10 grams of sulfolane (tetramethylene sulfone, Phillips Petroleum Co.) was loaded into a clean, dry 1000 cc autoclave. Sulfolane was stored in a vacuum desiccator over $P_2O_5$ prior to use. The autoclave was sealed and cooled with liquid nitrogen. The autoclave was evacuated and 40 grams of anhydrous HF (Matheson) were condensed into the autoclave. The HF/sulfolane mixture was warmed to room temperature (71°F, 22°C). Isobutane (100 grams) was added to the mixture, the autoclave was pressurized to 100 psig (0,79 MPa) and stirred at 1500 rpm. A pre-mixed 10/1 wt/wt isobutane/2-butene feed (Matheson) was then introduced at 250 cc/hr. A 5-10°F (2-5°C) temperature rise was typically observed during reaction. After two hours, feed addition was halted and a 300 cc liquid sample was obtained. The liquid sample was depressured through an ice cooled trap (filled with 50 cc of water) which was connected to a gas sampling bomb and wet test meter. The liquid alkylate product and gas sample were analyzed with a Varian 6000 gas chromatograph equipped with a 60 meter DB-1 capillary column.

Table A below lists the results with HF/sulfolane mixtures containing up to 50 wt% sulfolane in HF. With 20 wt% sulfolane in HF (125 moles HF per liter sulfolane), performance was comparable to pure HF. The ratio of high octane trimethyl-pentanes to lower octane dimethylhexanes (TMP/DMH) was 9.4 with 80/20 HF/sulfolane compared to 9.2 with pure HF. Performance diminished slightly upon adding 50 wt% sulfolane to HF (63 moles HF per liter of sulfolane). Alkylate with 50/50 HF/sulfolane catalyst had a TMP/DMH ratio of 6.5 and contained 11.8 wt% $C_9$+. A 40/60 HF/sulfolane catalyst (42 moles of HF per liter of sulfolane) showed no activity for alkylation. The only observed product was butyl fluoride formed by hydrofluorination of 2-butene feed. Thus, the useful-concentration range for sulfolane in HF was shown to be below about 60 wt% in HF (greater than about 40 moles of HF per liter of sulfolane).

HF/sulfolane catalyst performance is plotted as a function of sulfolane loading in Figures 1a, 1b, and lc. Alkylate quality increased slightly upon adding 20 wt% sulfolane to HF ($C_5$+ MON 97.5 vs 97 for pure HF), then decreased with further sulfolane dilution. Activity for isoparaffin/olefin alkylation was not observed above about 50 wt% sulfolane in HF.

Vapor pressures (Torr) for HF/sulfolane mixtures are plotted as a function of sulfolane loading in Figure 2, showing that diluting HF with sulfolane dramatically reduces vapor pressure.

Table A

| Example | 1 | 2 | 3 | 4 |
| --- | --- | --- | --- | --- |
| Catalyst | HF | HF/ Sulfolane (80/20) | HF/ Sulfolane (60/40) | HF/ Sulfolane (50/50) |
| Appearance | Fuming | Fuming | Liquid | Liquid |
| Alkylate Product wt % | | | | |
| $C_5$-$C_7$ | 5.5 | 4.7 | 5.9 | 8.3 |
| $C_8$ | 88.1 | 89.3 | 85.5 | 79.9 |
| $C_9$+ | 6.4 | 6.0 | 8.6 | 11.8 |
| TMP/DMH | 9.2 | 9.4 | 7.5 | 6.5 |
| Olefin Conv., % | 99.9 | 100 | 98.0 | 98.8 |

Examples 5-7

HF/sulfolane mixtures were evaluated in a 1.5 foot (46 cm) long riser reactor having total volume of about 120 cc. The reactor was first filled with liquid isobutane at approximately 130 psig (1 MPa). Approximately 100 cc of premixed HF/sulfolane catalyst (70/30 wt/wt for Example 5, 60/40 wt/wt for Examples 6 and 7) was pressured into the reactor from the bottom, displacing a portion of the isobutane. The isobutane flow was maintained to keep the injection nozzle clear. Hydrocarbon residence time in the riser was approximately 6 seconds with a hydrocarbon drop diameter of about 300 microns. The olefin feed was obtained from a commercial HF alkylation process unit and was characterized by the following average composition:

| Component | Weight Percent |
|---|---|
| $C_3$= | 12.4 |
| $C_4$= | 81.8 |
| $C_5$= | 5.8 |
| Total: | 100.0 |

Conversion conditions and results for Example 5 are shown below in Table B. The yields reported in Table B are given as g $C_5$+ alkylate per g olefin converted.

Table B

| Example | 5 | 6 | 7 |
|---|---|---|---|
| Temperature, °F | 90 (32°C) | 90 (32°C) | 90 (32°C) |
| Pressure, psig | 130 (1 MPa) | 125 (0,96 MPa) | 125 (0,96 MPa) |
| Isoparaffin:Olefin Ratio in the Reactor Feed | 17.1 | 14.9 | 14.3 |
| Recovery, wt % | 100.5 | 99.6 | 94.1 |
| Conversion, wt% | 98.2 | 98.9 | 99.2 |
| C8 TMP/DMH | 5.0 | 5.9 | 5.8 |
| $C_5$s, wt% | 10.1 | 5.1 | 4.9 |
| $C_6$s, wt% | 3.5 | 3.2 | 3.2 |
| $C_7$s, wt% | 10.8 | 3.7 | 3.7 |
| $C_8$s, wt% | 61.6 | 74.9 | 74.4 |
| $C_9^+$ | 12.9 | 12.5 | 13.2 |
| % TMP, wt% | 65.0 | 64.0 | 63.5 |
| Yield, wt basis | 2.2 | 2.0 | 2.1 |
| R+O, raw | 97.1 | 96.9 | 97.0 |
| M+O, raw | 95.2 | 95.2 | 95.3 |

<u>Examples 8-15</u>

Examples 8-15 examined the cloud-forming tendency of various mixtures of HF and sulfolane. The cloud-forming tendency was measured in terms of rainout, which is defined as the fraction of the released substance which falls to the ground within a defined area downwind from the point of release. For example, if 50% (weight) of a released substance falls to the ground within the defined area downwind from the point of release, the rainout is 50% (weight). Rainout is inversely related to cloud-forming tendency. In the event of an accidental release, material which precipitates or rains out cannot pose a toxic hazard downwind from the site of the accidental release.

The HF and sulfolane were mixed in a 2-liter stirred autoclave which was fabricated from 316 stainless steel and contained a cooling/heating coil. The autoclave was pressured with nitrogen, and the contents of the autoclave were then released through a nozzle into a flow chamber. The liquid rainout was collected in trays and the material which carried over was scrubbed with water in accordance with the procedure set forth below.

The sulfolane was weighed and placed into the 2-liter autoclave. Anhydrous HF from a cylinder was measured using a calibrated Kel-F lined sight glass and pressured into the autoclave. The amount of HF used per run varied from 109 to 434 grams. Once the material was loaded into the autoclave, the wind velocity in the flow chamber was adjusted to the desired setting, usually about 0.9 m/s to simulate a typical release wind speed. Ambient humidity was used. The autoclave mixer was also turned on for several minutes to mix the contents. Water was placed into each of three dropout trays to catch the precipitated HF and sulfolane as well as to minimize evaporative losses. The water-filled trays were weighed to determine the pre-release base line weights.

The autoclave pressure was then set with nitrogen and the autoclave temperature was set by warm water or a Neslab cooler. The valve to the release orifice was then opened, and the material flowed into the chamber. The orifice diameter was 0.635 mm. The dimensions of the flow chamber were approximately 40 in. (1m) (length) by 12 in. (0,3 m) (height) by 6 in. (0,15 m) (depth). Because the release material had sufficient inertia to travel a greater length than the flow chamber, an impingement plate was placed at the end of the third tray. This plate was covered with steel wool to minimize splashing and to produce rainout of 98 percent with the base case water runs.

The liquid rainout was collected in the trays, with most of the material going into the tray closest to the impingement plate. The trays were then weighed to determine how much material rained-out. The data were then corrected for evaporation of water from the trays, liquid hold-up in the impingement plate pad, residual material in the autoclave, and material that adhered to the walls of the chamber.

The rainout data are shown below in Table C, and plotted in Figure 3. Table C also lists the corresponding $C_5$+ alkylate TMP (trimethylpentane) content produced with each of the HF/sulfolane mixtures as isobutane:butene alkylation catalyst. These results show that the preferred catalyst composition provides excellent rainout (>70 wt.%) while also producing a high quality alkylate product.

Table C

| Conditions: about 50 psig (0,45 MPa), 90°F (32°C), 0.635 mm orifice diameter. | | | |
|---|---|---|---|
| Example No. | Sulfolane Wt % | TMP Wt % | Rainout, wt % by Material Balance |
| 8 | 60 | * | |
| 9 | 50 | | 76 |
| 10 | 50 | 69 | 76 |
| 11 | 50 | | 82 |
| 12 | 40 | 73 | 76 |
| 13 | 40 | | 78 |
| 14 | 30 | 78 | 81 |
| 15 | 20 | 80 | 32 |

* Less than 5 wt %.

Changes and modifications in the specifically described embodiments can be carried out without departing from the scope of the invention which is intended to be limited only by the scope of the appended claims.

## Claims

1. A catalyst composition comprising from 50 to 80 weight-% hydrofluoric acid and from 20 to 50 weight-% of a sulfone selected from a sulfone having the formula R-$SO_2$-R', wherein R and R' are the same or are different, and are selected from the group consisting of halides, alkyl groups, aromatic groups, alkylhalide groups, or arylhalide groups containing from 1 to 20 carbon atoms and a sulfone having the formula R-$SO_2$, wherein R is a bidentate alkyl group, an aromatic group, an alkylhalide group, or an arylhalide group containing from 1 to 20 carbon atoms.

2. The catalyst composition of claim 1, wherein said sulfone is sulfolane.

3. The catalyst composition of claim 2, wherein the catalyst composition comprises from 20 to 40 weight-% sulfolane and 60 to 80 weight-% hydrofluoric acid.

4. The catalyst composition of any of claims 1 to 3, wherein the catalyst composition comprises 20 to 30 weight-% sulfolane and 70 to 80 weight-% hydrofluoric acid.

5. The use of a catalyst composition of any of claims 1 to 4 in a isoparaffin-olefin alkylation process.

6. The use of claim 5, wherein the alkylation process temperature is from 50 to 150°F (+10 to 66 °C) and the alkylation process pressure is from 20 to 1200 psig (0.24 to 8.4 MPa).

7. The use of claim 5 or 6, wherein the molar ratio of isoparaffin to olefin is from 1:1 to 100:1.

8. The use of any of claims 5 to 7, wherein the molar ratio of isoparaffin to olefin is from 5:1 to 20:1.

9. The use of any of claims 5 to 8, wherein the feedstocks used in the isoparaffin-olefin alkylation process include at least one isoparaffin and at least one olefin, with the isoparaffin having from 4 to 8 carbon atoms and the olefin having from 2 to 12 carbon atoms.

**Patentansprüche**

1. Katalysatorzusammensetzung, enthaltend 50 bis 80 Gew.-% Fluorwasserstoffsäure und 20 bis 50 Gew.-% eines Sulfons, ausgewählt aus einem Sulfon der Formel $R-SO_2-R'$, wobei R und R' gleich oder verschieden sind und aus der Gruppe ausgewählt sind, bestehend aus Halogeniden, Alkylgruppen, aromatischen Gruppen, Alkylhalogenidgruppen oder Arylhalogenidgruppen, enthaltend 1 bis 20 Kohlenstoffatome, und einem Sulfon der Formel $R-SO_2$, wobei R eine zweizähnige Alkylgruppe, eine aromatische Gruppe, eine Alkylhalogenidgruppe oder eine Arylhalogenidgruppe, die 1 bis 20 Kohlenstoffatome enthält, bedeutet.

2. Katalysatorzusammensetzung nach Anspruch 1, wobei das Sulfon Sulfolan ist.

3. Katalysatorzusammensetzung nach Anspruch 2, wobei die Katalysatorzusammensetzung 20 bis 40 Gew.-% Sulfolan und 60 bis 80 Gew.-% Fluorwasserstoffsäure enthält.

4. Katalysatorzusammensetzung nach einem der Ansprüche 1 bis 3, wobei die Katalysatorzusammensetzung 20 bis 30 Gew.-% Sulfolan und 70 bis 80 Gew.-% Fluorwasserstoffsäure enthält.

5. Verwendung einer Katalysatorzusammensetzung nach einem der Ansprüche 1 bis 4 bei einem Isoparaffin-Olefin-Alkylierungsverfahren.

6. Verwendung nach Anspruch 5, wobei die Alkylierungsverfahrenstemperatur 50 bis 150 °F (+10 bis 66 °C) und der Alkylierungsverfahrensdruck 20 bis 1200 psig (0,24 bis 8,4 MPa) betragen.

7. Verwendung nach Anspruch 5 oder 6, wobei das Molverhältnis von Isoparaffin zu Olefin 1:1 bis 100:1 beträgt.

8. Verwendung nach einem der Ansprüche 5 bis 7, wobei das Molverhältnis von Isoparaffin zu Olefin 5:1 bis 20:1 beträgt.

9. Verwendung nach einem der Ansprüche 5 bis 8, wobei die bei dem Isoparaffin-Olefin-Alkylierungsverfahren verwendeten Ausgangsmaterialien mindestens ein Isoparaffin und mindestens ein Olefin enthalten, wobei das Isoparaffin 4 bis 8 Kohlenstoffatome und das Olefin 2 bis 12 Kohlenstoffatome aufweisen.

**Revendications**

1. Une composition de catalyseur comprenant 50 à 80 % en poids d'acide fluorhydrique et 20 à 50 % en poids d'une

sulfone choisie parmi une sulfone de formule R-$SO_2$-R', où R et R' sont identiques ou différents et sont choisis dans le groupe constitué des halogénures, des groupes alkyle, des groupes aromatiques, des groupes ou des groupes halogénure arylés contenant de 1 à 20 atomes de carbone, et une sulfone de formule R-$SO_2$, où R est un groupe bidentate alkylé, un groupe aromatique, un groupe halogénure alkylé ou un groupe halogénure arylé contenant de 1 à 20 atomes de carbone.

**2.** La composition de catalyseur de la revendication 1, où ladite sulfone est du sulfolane.

**3.** La composition de catalyseur de la revendication 2, où la composition de catalyseur comprend de 20 à 40 % en poids de sulfolane et 60 à 80 % en poids d'acide fluorhydrique.

**4.** La composition de catalyseur de l'une des revendications 1 à 3, où la composition de catalyseur comprend de 20 à 30 % en poids de sulfolane et 70 à 80 % en poids d'acide fluorhydrique.

**5.** L'utilisation d'une composition de catalyseur de l'une des revendications 1 à 4 dans un processus d'alkylation isoparaffine-oléfine.

**6.** L'utilisation de la revendication 5, où la température du processus d'alkylation est de 50 à 150 °F (+10 à 66 °C) et la pression du processus d'alkylation est de 20 à 1200 psig (0,24 à 8,4 MPa).

**7.** L'utilisation de la revendication 5 ou 6, où le rapport molaire de l'isoparaffine à l'oléfine est de 1:1 à 100:1.

**8.** L'utilisation de l'une des revendications 5 à 7, où le rapport molaire de l'isoparaffine à l'oléfine est de 5:1 à 20:1.

**9.** L'utilisation de l'une des revendications 5 à 8, où les charges d'alimentation utilisées dans le processus d'alkylation isoparaffine-oléfine comprennent au moins une isoparaffine et au moins une oléfine, l'isoparaffine ayant de 4 à 8 atomes de carbone et l'oléfine ayant de 2 à 12 atomes de carbone.

Fig.1

PERFORMANCE OF HF/SULFOLANE MIXTURES

Fig.1a

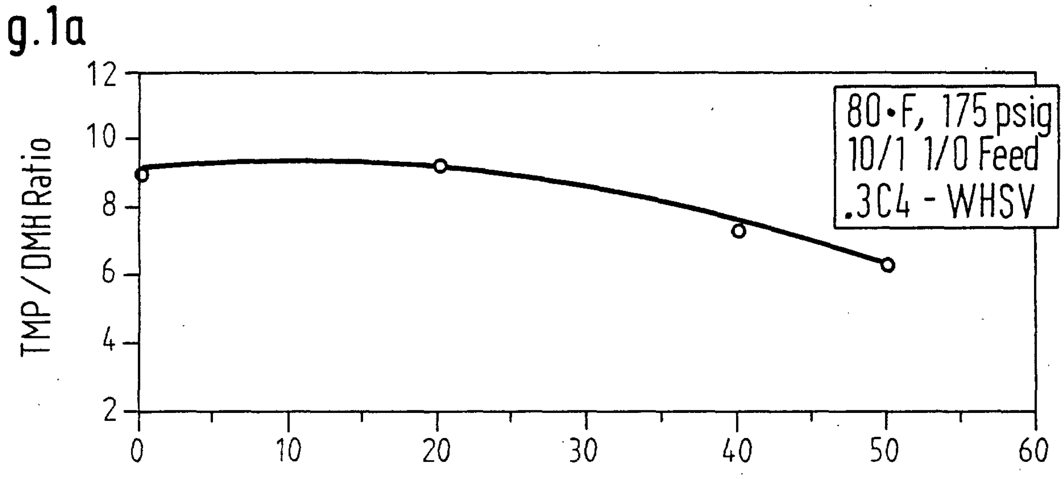


Fig.1b

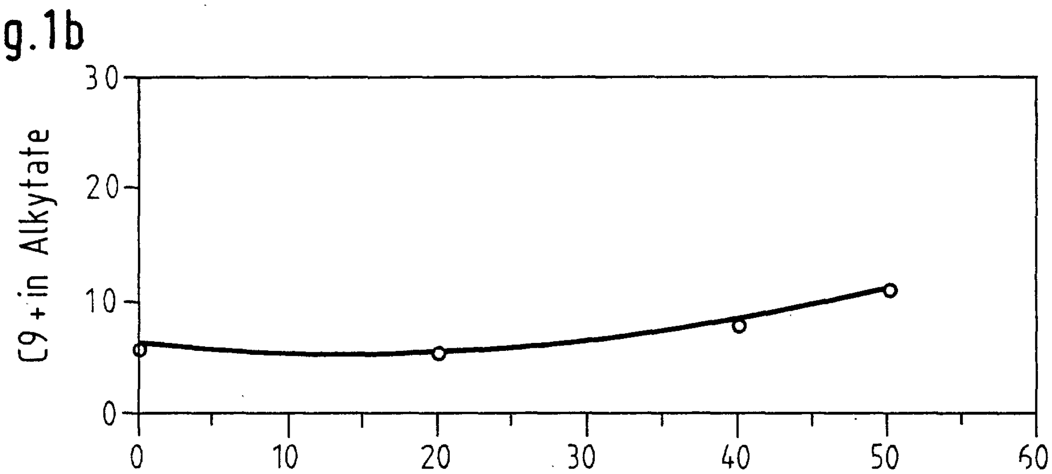


Fig.1c

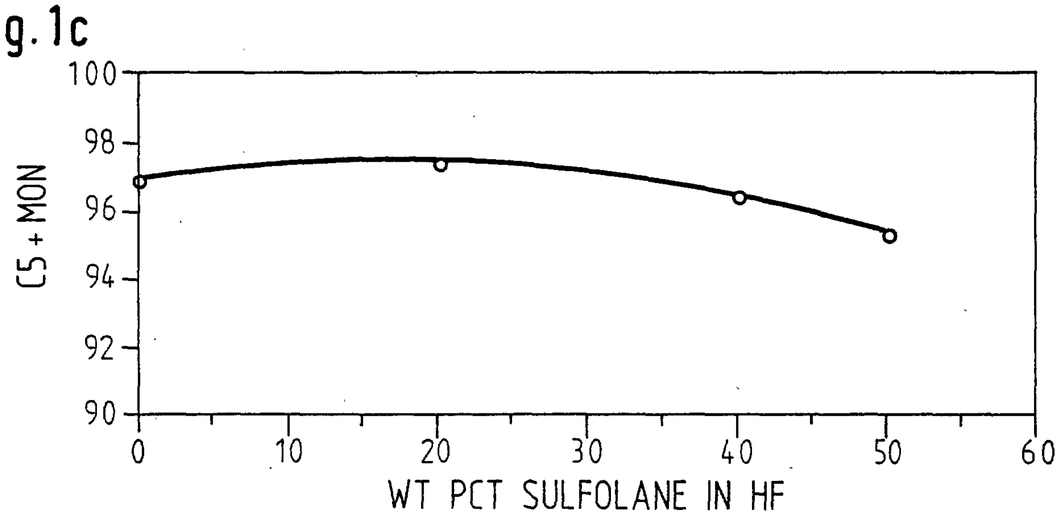

Figure 2

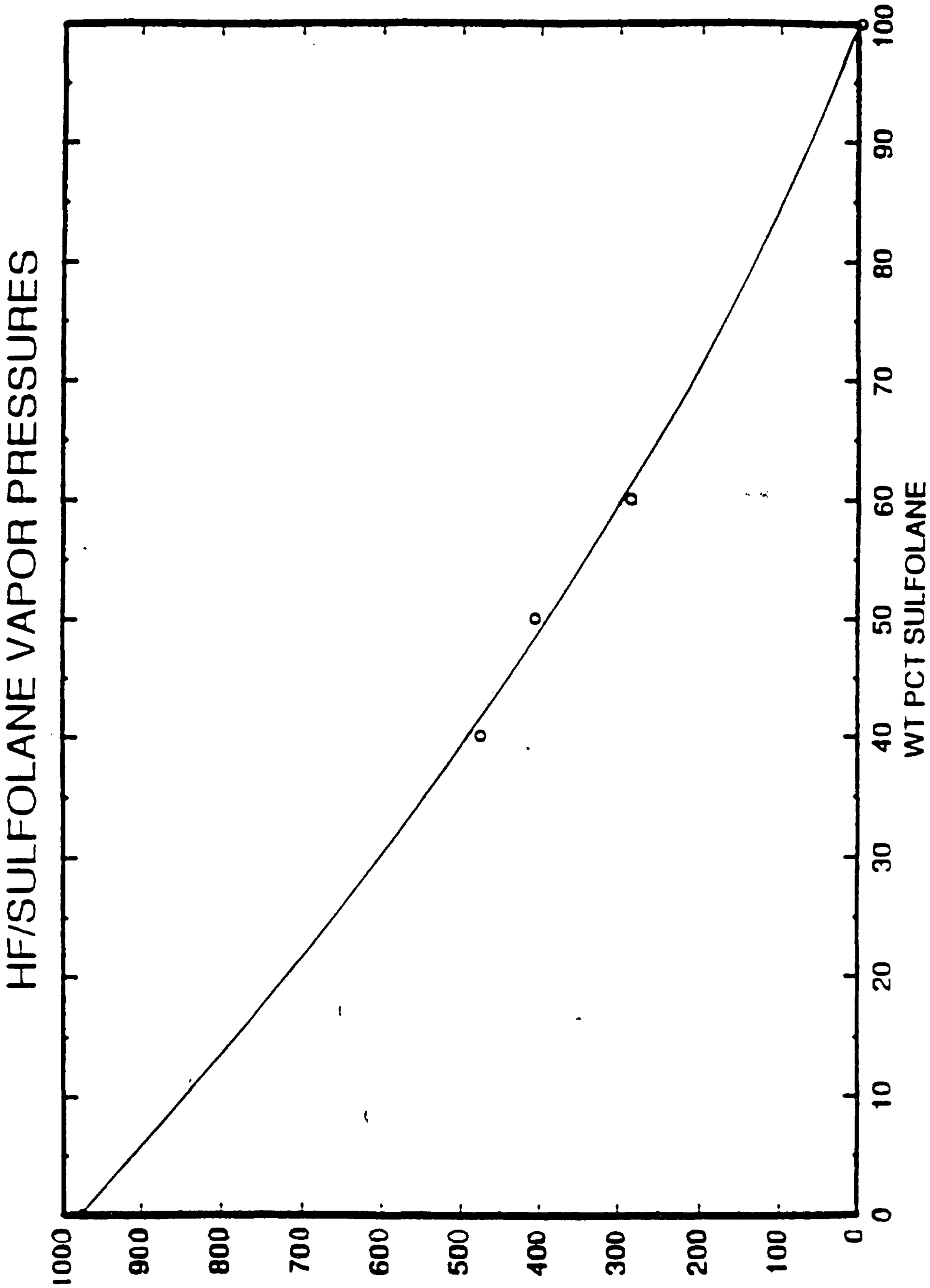
HF/SULFOLANE VAPOR PRESSURES
WT PCT SULFOLANE
0
10
20
30
40
50
60
70
80
90
100
0
100
200
300
400
500
600
700
800
900
1000

Fig. 3

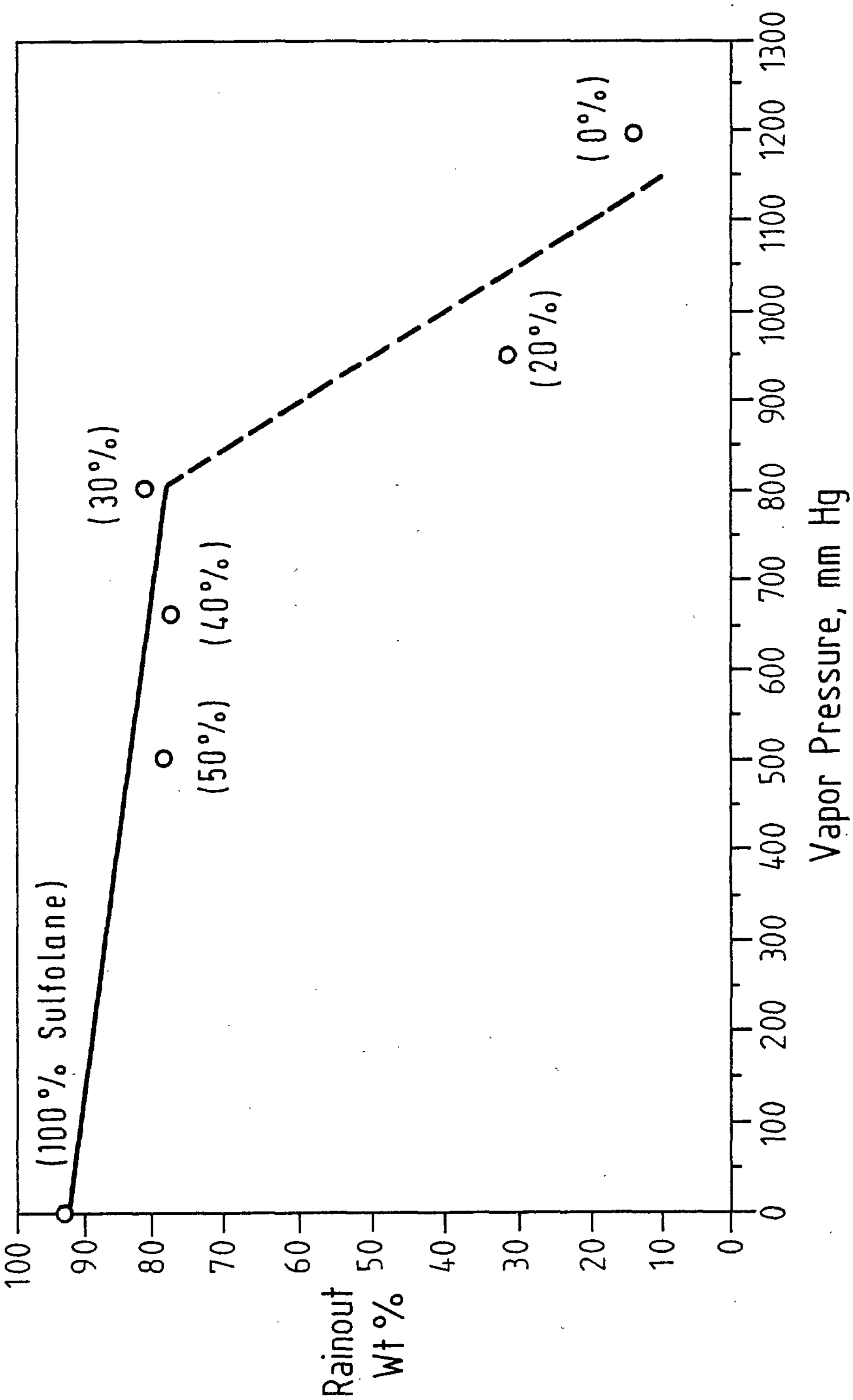
(100% Sulfolane)
(50%)
(40%)
(30%)
(20%)
(0%)
0 100 200 300 400 500 600 700 800 900 1000 1100 1200 1300
Vapor Pressure, mm Hg
0 10 20 30 40 50 60 70 80 90 100
Rainout Wt%